# EUROPEAN PATENT APPLICATION

(11) **EP 4 057 298 A1**
(43) Date of publication of application: **14.09.2022**
(21) Application number: 22160470.5
(22) Date of filing: 07.03.2022
(51) Int. Cl.: G16H 50/80, G16H 40/20

(54) **SYSTEM FOR AGGREGATING MULTI-SOURCE HEALTH-BASED SAFETY INFORMATION, METHOD THEREFOR, AND STORAGE MEDIUM**

(30) Priority: 10.03.2021 US 202117198136
(71) Applicant: Ricoh Company, Ltd., Tokyo 143-8555 (JP)
(72) Inventor: NUGGEHALLI, Jayasimha, Boulder, 80301-9270 (US); WATANABE, Kaoru, Boulder, 80301-9270 (US); RAMIREZ, Gerardo, Boulder, 80301-9270 (US); TANAKA, Shun, Tokyo, 143-8555 (JP)
(74) Representative: SSM Sandmair

(57) **Abstract**

A graphical user interface (GUI) is presented, which visually aggregates information from various information source systems to facilitate management of health-based safety practices in the workplace. Specifically, the methods and systems described herein visually aggregate information from at least a location information system and a health kiosk information system in a GUI. Specifically, a visual aggregation application includes, in a GUI, a map of a monitored physical area that displays health-interaction values for respective humans, associated with displayed location indicators, occupying the monitored physical area. According to an embodiment, the application includes, in the GUI, alerts of enhanced-risk distancing criteria violations in the monitored physical area. An enhanced-risk distancing criteria violation is an instance of distancing criteria violation between two or more humans in the monitored physical area, where one or more of the humans is also associated with a health warning.

## Description

### TECHNICAL FIELD

Embodiments relate to graphical user interfaces, and more specifically, to aggregating health-based workplace safety information from multiple information sources in a graphical user interface.

### BACKGROUND ART

The approaches described in this section are approaches that could be pursued, but not necessarily approaches that have been previously conceived or pursued. Therefore, unless otherwise indicated, it should not be assumed that any of the approaches described in this section qualify as prior art merely by virtue of their inclusion in this section.

For many kinds of businesses, it is important to gather at a dedicated office location (the "workplace") in order to conduct business activities. However, it can be difficult to maintain social distancing and other health-based safety practices while in the workplace. It is even more difficult for administrators and human resources departments to enforce health-based safety practices in the workplace.

In order to manage health-based safety practices in a workplace, it is important to gather and maintain accurate information regarding workplace practices and facilities. There are various information systems that can be used to gather such information, including health kiosks, workplace location monitors, and access management systems. However, these information systems gather information using a variety of techniques and data storage solutions, and accessing the information via the various systems can be cumbersome. For example, each system implemented in a given workplace is associated with its own interface to deliver information gathered by the system, and any information aggregation between systems is generally performed by the workplace administrators themselves. This kind of aggregation by hand is time-consuming and may be prone to error, depending on the method of aggregation.

It would be desirable and beneficial to automatically aggregate the information from multiple different information systems implemented within a given workplace in order to facilitate management of health-based safety practices in the workplace.

### SUMMARY

According to an embodiment, a computing system comprises one or more processors, and one or more memories storing one or more sequences of instructions. Processing of the instructions by the one or more processors causes receiving data from a set of information sources comprising a location information system and a health kiosk information system, wherein said receiving data comprises, for each information source of the set of information sources, receiving data via an application programming interface (API) for said each information source. Graphical user interface data is generated that, when processed at a computing device, causes display of a graphical user interface that visually aggregates representations of the received data from the set of information sources. The graphical user interface data is caused to be transmitted to a client computing device.

According to an embodiment, the received data comprises location data comprising one or more locations for one or more respective humans within the monitored physical area, and health-interaction data comprising one or more health-interaction values for each human of the one or more humans. Further, the graphical user interface comprises a map depiction of the monitored physical area with one or more location identifiers of the one or more humans, wherein locations, on the map depiction of the monitored physical area, of the one or more location identifiers are determined based on the location data. In this embodiment, each location identifier, of the one or more location identifiers, is visually associated with one or more displayed health-interaction values, of the health-interaction data, for a human associated with said each location identifier. According to an embodiment, a health status indicator from a plurality of health-interaction values, of the health-interaction data, for each human of the one or more humans, where said one or more displayed health-interaction values visually associated with each location identifier of the one or more location identifiers comprises the health status indicator determined for the human associated with said each location identifier.

According to an embodiment, the received data comprises location information for a plurality of humans in a monitored physical area, and health-interaction data comprising one or more health-interaction values of each human of the plurality of humans. In this embodiment, it is determined that the distancing criteria violation is an enhanced-risk distancing criteria violation based on determining that one or more humans, of the two or more humans, satisfy health warning criteria, wherein the graphical user interface comprises an alert of the enhanced-risk distancing criteria violation. According to an embodiment, the received data includes location data; and the graphical user interface further comprises a map depiction of the monitored physical area with a plurality of location identifiers of the plurality of humans, wherein locations, on the map depiction of the monitored physical area, of the plurality of location identifiers are determined based on the location data. In this embodiment, the alert comprises a visual indication of the enhanced-risk distancing criteria violation that is visually associated with the location identifiers of the two or more humans.

According to an embodiment the distancing criteria comprises a minimum distance to be maintained between humans and a maximum amount of time that the minimum distance may be breached before the distancing criteria is violated. According to an embodiment, determining that a particular human, of the one or more humans, satisfies the health warning criteria is based on one or more of: a body temperature of the particular human surpassing a temperature threshold; an illness diagnosis of the particular human; a reported red-flag symptom for the particular human; a plurality of previous distancing criteria violations involving the particular human; or a previous distancing criteria violation involving both (a) the particular human, and (b) another human with an illness diagnosis that occurred within a threshold amount of time of a timestamp associated with the previous distancing criteria violation. The approach may be implemented by one or more non-transitory computer-readable media storing instructions that, when processed by one or more processors, cause the approach to be implemented, or by one or more computer-implemented methods.

### BRIEF DESCRIPTION OF THE DRAWINGS

In the drawings:
FIG. 1 depicts an example computer system arrangement upon which embodiments may be implemented.
FIG. 2 is a flowchart for visually aggregating, in a graphical user interface, data from multiple information sources including a location system and a health kiosk system.
FIGS. 3A-3B depict an example workplace that is configured with sensors that are part of an example location system that monitors a workplace.
FIG. 4 depicts an example graphical user interface that includes a map of a monitored physical area, where the map displays location indicators for humans in the monitored
   physical area as well as health-interaction values for the humans.
FIGS. 5A-5B depict graphical user interfaces depicting elevated-risk distancing criteria violations for humans within the monitored physical area.
FIGS. 6A-6B depict graphical user interfaces that visually aggregate information from multiple information sources via graph/chart.
FIG. 7 depicts contents of an example workplace health data repository.
FIG. 8 is a block diagram that depicts an example computer system upon which embodiments may be implemented.

While each of the drawing figures depicts a particular embodiment for purposes of depicting a clear example, other embodiments may omit, add to, reorder, and/or modify any of the elements shown in the drawing figures. For purposes of depicting clear examples, one or more embodiments may be described with reference to one or more of the figures. However, using the particular arrangement depicted in the one or more figures is not required for the embodiments.

### DETAILED DESCRIPTION

In the following description, for the purposes of explanation, numerous specific details are set forth in order to provide a thorough understanding of the present disclosure. It will be apparent, however, that the present disclosure may be practiced without these specific details. In other instances, well-known structures and devices are shown in block diagram form in order to avoid unnecessarily obscuring the present disclosure. Modifiers such as "first" and "second" may be used to differentiate elements, but the modifiers do not necessarily indicate any particular order.
I. Overview
II. Health-Based Safety Visual Aggregation System
   A. Health Kiosk System
   B. Location System
   C. Geofencing System
   D. Workplace Management System
   E. Visitor Tracking System
   F. Access Management System
III. Graphical User Interface with Aggregated Health-Based Safety Information
   A. Graphical User Interface with a Map Displaying Health-Interaction Data
      i. Health Warning Criteria
      ii. Health Status Indicator
   B. Graphical User Interface with Elevated-Risk Distancing Criteria Violation Data
   C. Contact Tracing
   D. Graphical User Interface with Composite Statistics
IV. Workplace Health Data Repository
   A. Identity Information
   B. Location Information
   C. Aggregation based on Identifiers in Various Datasets
V. Architecture
VI. Implementation Examples

### I. Overview

A graphical user interface (GUI) visually aggregates information from various information source systems to facilitate management of health-based safety practices in the workplace. Specifically, the methods and systems described herein visually aggregate information from at least a location information system and a health kiosk information system in a GUI. Thus, the described GUI both provides summary information for high-level decisions, and also provides cross-source data analysis that incorporates information from the multiple sources. The automatic visual aggregation of health-based safety information reduces the number of applications required to be run by workplace administrators, thereby reducing the processing power required to manage health-based safety practices in the workplace. Also, use of the described GUI increases the efficiency of workplace administrators as they enforce health-based safety practices in the workplace.

### II. Health-Based Safety Visual Aggregation System

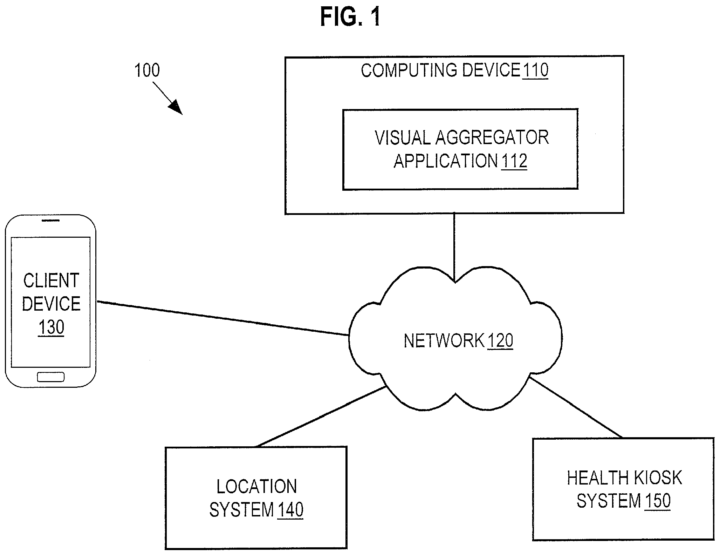

FIG. 1 depicts an example computing system arrangement 100 upon which embodiments may be implemented. Specifically, arrangement 100 of FIG. 1 includes a computing device 110 configured with a visual aggregator application (VAA) 112. Arrangement 100 also includes a client device 130, a location system 140, and a health kiosk system 150 communicatively coupled via network 120. VAA 112 is configured to generate data for a graphical user interface that visually aggregates data from information sources comprising location system 140 and health kiosk system 150, which are described in further detail below.

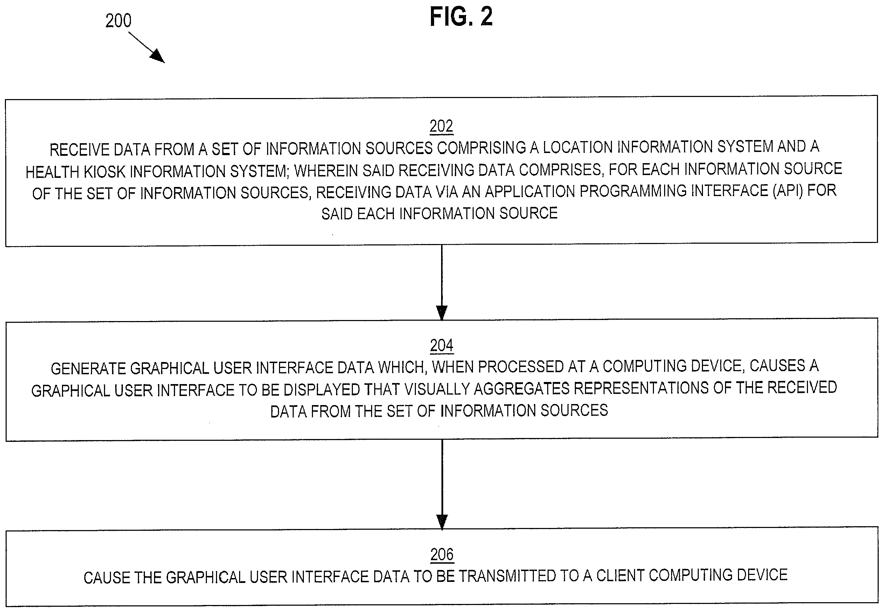

FIG. 2 is a flowchart 200 for generating data for a graphical user interface that visually aggregates data from multiple information sources. Specifically, at step 202 of flowchart 200, data is received, from a set of information sources comprising a location information system and a health kiosk information system, where receiving data comprises, for each information source of the set of information sources, receiving data via an application programming interface (API) for said each information source.

For example, VAA 112 at computing device 110 receives data, from at least location system 140 and health kiosk system 150, via an API for each system. An API is a computing interface that allows for communication between disparate entities by defining a communication protocol for the entities. An API may be used to request information from an information source, and may also be used to request that particular actions be taken by the information source system.

In an embodiment, VAA 112 is communicatively coupled to any of a number of external data sources that may or may not be represented in FIG. 1, such as a geofencing system, a workplace management system, a visitor tracking system, an access management system, etc. VAA 112 may be communicatively coupled to the external data sources via network 120. The external data sources may provide VAA 112 with access to any of a variety of data, workplace health-related or otherwise.

### A. Health Kiosk System

A health kiosk is a physical device, typically placed at entry points of a building/monitored physical area, that provides one or more health-related services. These one or more health-related services may include detection of body temperature, dispensing of hand sanitizer, scanning of codes (e.g., on an identification card), display of survey questions, input detection (e.g., a touch pad, keyboard, camera or other sensor to receive user input), facial scanning (e.g., to identify a person, to determine whether the person is wearing a mask), etc. Many times, a health kiosk provides touch-free services to avoid the need for frequent sanitization.

Health kiosk system 150 comprises one or more computing devices and one or more health kiosks communicatively coupled to computing device 110 via network 120. The computing devices of health kiosk system 150 may be distinct from the health kiosks of the system, and/or health kiosks of system 150 may have one or more integrated computing devices.

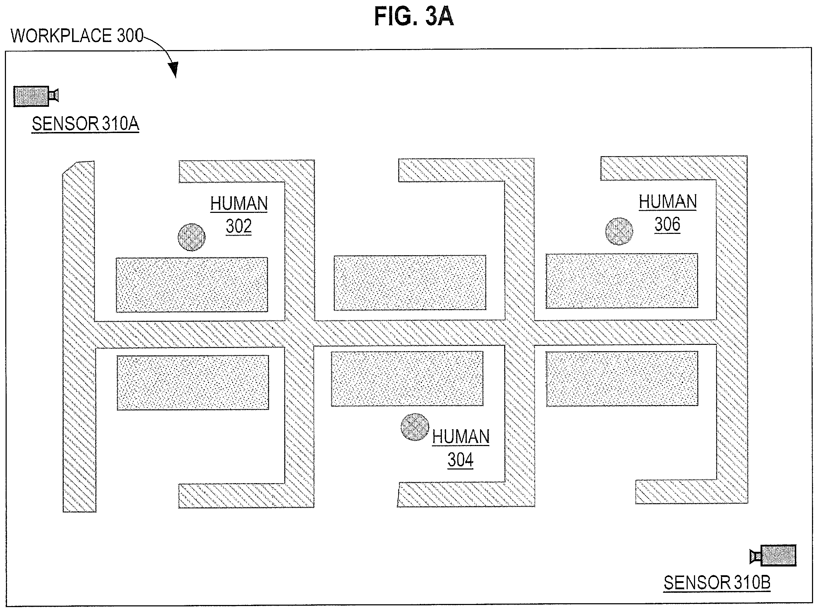

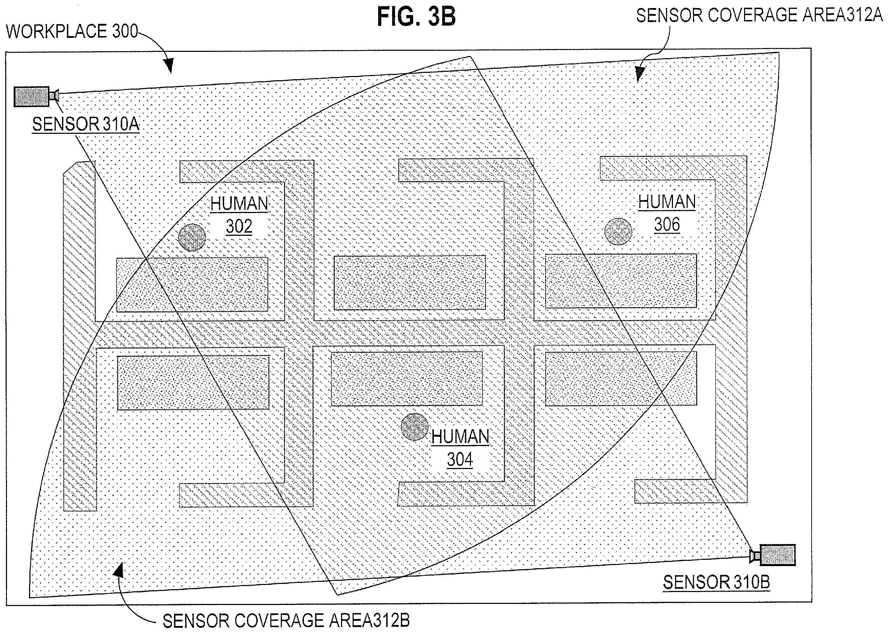

The one or more health kiosks of health kiosk system 150 are configured to collect health-interaction data for humans entering or occupying a monitored physical area (such as workplace 300 of FIG. 3). According to an embodiment, health kiosk system 150 maintains historical data indicating data collected during interactions with humans and/or transmits information indicating collected health-interaction information to VAA 112. As used herein, health-interaction data refers to any health- or workplace safety-related data collected by an information source, such as health kiosk system 150. The health-interaction data collected by health kiosk system 150 includes one or more of: a timestamp of kiosk interaction, body temperature, identifier scan results, one or more survey answers, facial recognition results, voice recognition results, one or more images, one or more voice recordings, mask detection results, sanitizer dispensing results, self-reported illness diagnosis, etc.

According to an embodiment, health kiosk system 150 provides, to VAA 112, information to support enforcement of health-based entrance criteria for humans entering the monitored physical area, such as an identification requirement, a maximum body temperature, wearing of a mask, and particular answers to health survey questions. In this embodiment, VAA 112uses the entrance criteria to determine whether a particular human should be allowed within the monitored physical area. According to an embodiment, VAA 112 causes display, via a display device (e.g., at a health kiosk of health kiosk system 150), whether a user is cleared for entry into the monitored physical area. According to an embodiment, VAA 112 sends a message indicating whether a given human is cleared for entry into the monitored physical area to another system (such as an access management system, e.g., a key card entrance system that controls a locked entrance door into the monitored physical area as described in further detail below) or to a guard that allows admittance to the monitored physical area.

Health kiosk system 150 includes an API through which VAA 112 may communicate with system 150. For example, via the health kiosk system API, VAA 112 may request periodic updates of health-interaction information, real-time updates of health-interaction information, real-time notifications of detected entrance criteria violations, historical data indicating entrance criteria violations, etc. Health kiosk system 150 provides the requested information to VAA 112 via network 120, either in real-time or periodically.

### B. Location System

Location system 140 provides location information for people/objects within a monitored area. The location information provided by location system 140 may be real-time information. As used herein, a location for a person refers to a location that is associated with the person, which may be a reserved location, a workspace location, a real-time location, an entrance location, etc. Location system 140 may be implemented in many ways, including by one or more of a geofencing system, a visitor tracking system, a workplace management system, an access management system, etc., or by any combination of such systems.

### C. Geofencing System

A geofence is a virtual perimeter for a physical area. A geofencing system, which is described herein as at least partially implementing example location system 140, comprises one or more computing devices that are configured to detect real-time locations of humans within a virtual perimeter that defines the boundaries of a monitored physical area, such as the workplace. A geofencing system may be implemented in any way that allows for real-time tracking of human locations within the monitored physical area.

According to an embodiment, a geofencing system is implemented, at least partly, using location-aware devices worn by, carried by, or attached to humans within the monitored physical area. A location-aware device is a device that tracks the geographic location of the device and transmits (in real-time or at more lengthy intervals) location information to a geofencing system. Examples of location-aware devices include, without limitation, wearable computing devices (such as smart watches, eyeglass computing devices, implantable computing devices, etc.) and portable computing devices (such as smart phones, personal digital assistants, tablet computing devices, laptop computers, etc.). In this embodiment, a geofencing system compiles the transmitted data from the location-aware devices to track the locations of the devices (and, accordingly, locations of the associated humans) within the monitored physical area.

According to an embodiment, a geofencing system is implemented, at least partly, using identification devices worn/carried by or attached to humans within the monitored physical area. An identification device is a device that allows one or more sensors of a geofencing system to track the geographic location of the device. Examples of identification devices include radio frequency identification (RFID) tags, identification (ID) cards, infrared (IR) beacon-based devices, etc. In this embodiment, a geofencing system uses one or more sensors (such as IR receiver sensors, cameras, etc.) to identify the locations of the identification devices within the monitored physical area to track the locations of the associated humans within the monitored physical area.

According to an embodiment, a geofencing system is implemented, at least partly, using one or more sensors that are communicatively connected to one or more computing devices configured to detect humans within the monitored physical area. For example, a geofencing system uses camera-based sensors and one or more computer vision applications to detect and/or identify the humans within the monitored physical area. As another example, a geofencing system uses microphone-based sensors and one or more sound-based applications to locate and/or identify and/or confirm identities of the humans within the monitored physical area. Voice recognition may be used in combination with face/body recognition to identify humans. As yet another example, a geofencing system uses Global Positioning System (GPS) data to detect the locations of humans within the monitored physical area. As a further example, a geofencing system uses proximity sensors (such as sound-based sensors, light-based sensors, IR-based sensors, electromagnetic field-based sensors, touch sensors, etc.) to locate objects and humans within the monitored physical area.

To illustrate an example configuration for a geofencing system involving one or more sensors, FIGS. 3A-3B depict an example workplace 300 that is configured with example sensors 310A and 310B that are part of a geofencing system that monitors workplace 300. In the example configuration of workplace 300, three humans 302-306 inhabit staggered cubicles to maintain a minimum distance, according to distancing criteria described in further detail below.

Sensors 310A and 310B are depicted as being placed in opposite corners of example workplace 300, but embodiments are not limited to this example and are applicable to any number of sensors in any locations. As shown in FIG. 3B, the sensor coverage areas 312A and 312B of the respective sensors 310A and 310B cover the majority of workplace 300. According to an embodiment, the sensor coverage areas of sensors of a geofencing system cover a portion of, a majority of, or all of the monitored physical area. Sensors 310A and 310B are depicted as cameras, but embodiments described herein are not limited thereto, as described in detail above.

According to an embodiment, a geofencing system maintains distancing criteria for the human locations, such as a minimum distance to be maintained between humans (such as six feet) and a maximum amount of time that the minimum distance may be breached before the distancing criteria is violated (such as 15 minutes). As another example of distancing criteria, a maximum number of people for a given room of the monitored physical area is not exceeded for more than a maximum amount of time. For example, a room that has a capacity of four people is registered (e.g., through a booking function of a visitor tracking system or workplace management system) as having five or more people occupying the room for more than 15 minutes. Detection of a number of people in a room may be determined based on a room-specific access management system, e.g., that requires or performs user identification verification for room entrance/exit.

In this embodiment, a geofencing system uses the maintained distancing criteria to determine whether the gathered location information indicates that any humans have violated the distancing criteria. For example, two humans that are determined to be less than a minimum distance apart for more than the maximum amount of time, as specified by the distancing criteria, are considered to have violated the distancing criteria. A geofencing system maintains historical data indicating violations of distancing criteria and/or transmits information indicating violations of distancing criteria to VAA 112.

A geofencing system includes an API through which VAA 112 may communicate with one or more computing devices of the geofencing system that are communicatively coupled to computing device 110 via network 120. For example, via the location system API, VAA 112 may request periodic updates of human locations, real-time updates of human locations, real-time notifications of detected distancing criteria violations, historical data indicating historical distancing criteria violations, etc. A geofencing system provides the requested information to VAA 112 via network 120, either in real-time or periodically. Real-time processing and/or communication of information means processing or communication that happens within a short time span of the events reflected in the information that is being processed or communicated.

### D. Workplace Management System

A workplace management system automatically manages one or more aspects of the workplace, including one or more of: desk/workspace management, return to work policy/enforcement, wayfinding, service requests, room management, Internet of Things (IoT) management, digital signage, etc. A workplace management system comprises one or more computing devices communicatively coupled to computing device 110 via network 120, or may be implemented as a cloud-based application.

According to an embodiment, location system 140 is at least partially implemented by a workplace management system. In this embodiment, the workplace management system records locations for persons within the monitored physical area based on work areas assigned to the person, rooms booked for the person, user log-ins or check-ins at computing devices that are registered with the workplace management system, destination or origination locations of wayfinding requests, etc. Furthermore, a workplace management system may collect other kinds of information that VAA 112 visually aggregates with one or more other information systems.

Such a workplace management system includes an API through which VAA 112 may communicate with the system. For example, via the workplace management system API, VAA 112 may request periodic or real-time updates of workplace management system information. The system provides the requested information to VAA 112 via network 120, either in real-time or periodically.

### E. Visitor Tracking System

A visitor tracking system automatically manages visitors to a monitored physical area. For example, through a visitor tracking system, a host (e.g., an employee at the workplace) provides information identifying a new visitor and the visitor tracking system assigns, to the visitor, a visitor ID. The host may also schedule a visit for the visitor, reserve a room for the visitor, and/or send an invitation to the visitor (e.g., via email, text, telephone, etc.).

Once the visitor arrives at the workplace, the visitor checks in with the visitor tracking system. For example, the visitor provides identifying information (such as a visitor Quick Response (QR) code, a verbal confirmation of user identification, an image of the visitor's face for a facial recognition application, etc.). The visitor may provide this information in any manner, e.g., via a GUI for the visitor tracking system, via a health kiosk, via an access management device, via an access management GUI, etc. For example, the visitor provides information via a health kiosk, and health kiosk system 150 provides the received information to the visitor tracking system. The registered host receives a notification from the visitor tracking system that the visitor has checked in.

According to an embodiment, the visitor tracking system further provides to the visitor information needed for the visit, e.g., via a GUI for the visitor tracking system. This information may include connection information for a guest Wi-Fi service, information for guest printing and scanning for on-premises devices, connection information for one or more interactive whiteboards, etc.

After the visit is completed, the visitor checks out of the visitor tracking system in any way, e.g., via a GUI for the visitor tracking system, via an access management system, etc. Once checked out, a status of the visitor, maintained by the visitor tracking system, is changed from "checked-in" to "checked-out".

According to an embodiment, location system 140 is at least partially implemented by a visitor tracking system. In this embodiment, the visitor tracking system records locations for persons within the monitored physical area based on location information associated with visitor check-ins and check-outs (e.g., the locations of the access management devices used for visitor check-in or check-out), based on rooms booked for use by the visitor, based on visitor use of devices within the monitored physical area (such as printers, scanners) etc. Furthermore, a visitor tracking system may collect other kinds of information that VAA 112 visually aggregates with information from one or more other information systems.

A visitor tracking system comprises one or more computing devices communicatively coupled to computing device 110 via network 120, or may be implemented as a cloud-based application. Such a visitor tracking system includes an API through which VAA 112 may communicate with the system. For example, via the visitor tracking system API, VAA 112 may request periodic or real-time updates of information gathered by the visitor tracking system. The system provides the requested information to VAA 112 via network 120, either in real-time or periodically.

### F. Access Management System

An access management system automatically manages entrance to a monitored physical area. An access management system comprises one or more computing devices communicatively coupled to computing device 110 via network 120, or may be implemented, at least in part, as a cloud-based application.

According to an embodiment, an access management system further comprises one or more access devices that gather information from people that attempt to enter the monitored physical area. For example, an access management system comprises one or more scanners that are configured to read employee and/or visitor identification, e.g., by scanning a bar code or QR code, by reading a magnetic strip on a card, by taking an image of a person or an identification card and using computer vision to extract information from the image, etc. According to an embodiment, the access management system further comprises locking mechanisms that the system automatically releases when the information gathered from a proposed entrant to the monitored physical area matches information on a grant-access list maintained by the system.

According to another embodiment, the access management system is run by one or more human guards that check identity information and use the access management system to grant or deny access to the human requesting entrance. In this embodiment, the access management system automatically allows access to authorized entrants via one or more locking devices.

According to an embodiment, an access management system receives input for a person requesting entrance to the monitored physical area from health kiosks of health kiosk system 150, including a user identifier and health-based authorization to enter the monitored physical area. Based on the health-based authorization to enter the premises, the access management system checks the user identifier against a grant-access list. If the user identifier is in the grant-access list, the access management system allows entrance to the person, e.g., by automatically disengaging a locking mechanism for a door that is near the health kiosk being used by the entrant.

According to an embodiment, an access management system identifies people when leaving the premises. For example, the system identifies people leaving the premises based on scanning a user ID card, performing facial recognition based on images taken of the people nearing the exit, performing voice recognition based on recordings taken of people nearing the exit, etc.

According to an embodiment, location system 140 is at least partially implemented by an access management system. In this embodiment, the access management system records locations for persons within the monitored physical area based on entrance locations used, timestamps of entrance requests, images taken of entrants, whether an entrance request was accepted or denied. An access management system may also gather such information for people exiting the premises.

Furthermore, an access management system may collect of information that VAA 112 visually aggregates with one or more other information systems. For example, in a map-style GUI reflecting location information for a person, VAA 112 causes the location indicator for the person to be associated with a listing of one or more access management statistics, such as the number of times access to the monitored physical area has been denied to the person within a given timeframe. According to an embodiment, VAA 112 determines that a particular person satisfies access warning criteria, such as having more than three denied entrances within the last month. In response to this determination, VAA 112 automatically associates a location indicator for the person with a visual warning indicator, such as a style or color of access management statistics text, a color of the location indicator, an icon that is visually associated with the location indicator, etc.

Such an access management system includes an API through which VAA 112 may communicate with the system. For example, via the access management system API, VAA 112 may request periodic or real-time updates of access management system information. The system provides the requested information to VAA 112 via network 120, either in real-time or periodically.

### III. Graphical User Interface with Aggregated Health-Based Safety Information

At step 204 of flowchart 200, graphical user interface data is generated such that the graphical user interface data being processed at a computing device causes a graphical user interface to be displayed that visually aggregates representations of the received data from the set of information sources. The graphical user interface data may be any type or in any form that may vary depending upon a particular implementation. For example, graphical user interface data may be application data or one or more Web pages. VAA 112 receives updates of human location information from location system 140 and updates of health-interaction information from health kiosk system 150 and stores the information in a workplace health data repository, described in further detail below. VAA 112 receives a request, from client device 130, to display a GUI with visually aggregated information from location system 140 and health kiosk system 150. In response to receiving the request, VAA 112 generates GUI data that, when processed client device 130, causes a graphical user interface to be displayed at a display device of client device 130. According to embodiments described herein, the graphical user interface visually aggregates information from location system 140 and health kiosk system 150 stored within workplace health data repository.

At step 206 of flowchart 200, the graphical user interface data is caused to be transmitted to a client computing device. Continuing with the example above, VAA 112 sends the generated information for the GUI to client device 130. In response to receiving the GUI information, client device 130 processes the GUI information, which causes display of the GUI on a display device of client device 130. To illustrate, the GUI information is processed by a health aggregator client application running on client device 130 that displays the GUI, or is processed by a browser running on client device 130 that displays the GUI as a webpage. As another example, the client device 130 may include a client application that implements an API supported by the VAA 112 and the client application queries the VAA 112. The VAA 112 provides the graphical user interface data to the client device 130 in response to the queries. Thus, the graphical user interface data may be pushed by the VAA 112 to the client device 130 or pulled by the client device 130 from the VAA 112.

### A. Graphical User Interface with a Map Displaying Health-Interaction Data

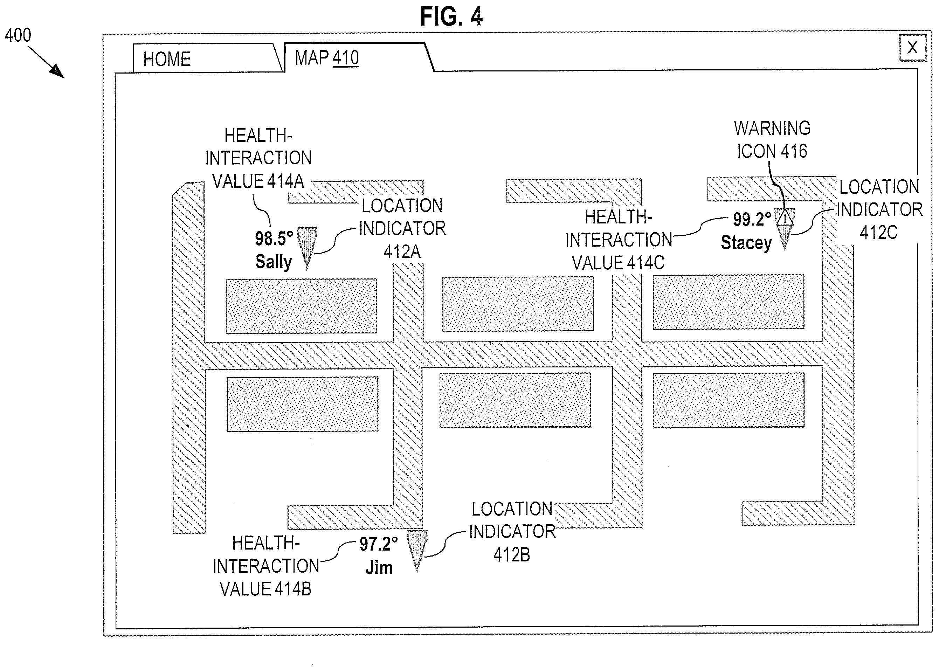

FIG. 4 depicts an example GUI 400 that includes a map 410 of a monitored physical area that displays health-interaction values 414A-C for respective humans, associated with displayed location indicators 412A-C, occupying the monitored physical area. According to an embodiment, map 410 is a "real-time" map that is updated based on real-time location and/or health interaction data. The example health-interaction values retrieved from system 150 and depicted in GUI 400 are the latest body temperature readings for the associated humans, however, embodiments are not limited to depicting body temperature readings and may additionally or alternatively display any of the collected health-interaction data stored for the humans.

According to an embodiment, VAA 112 receives data, from location system 140, that includes location data comprising one or more locations for one or more respective humans within the monitored physical area. VAA 112 also receives health-interaction data, from health kiosk system 150, comprising one or more health-interaction values for each human of the one or more humans.

VAA 112 includes, in the graphical user interface, a map depiction of a monitored physical area (such as map 410 of GUI 400). Furthermore, based on the location data, VAA 112 populates the map depiction, of the graphical user interface, with one or more location identifiers for the one or more respective humans. For example, VAA 112 determines locations for location indicators 412A-412C based on the most recent location values in the received location data. According to an embodiment, as VAA 112 receives real-time updates for locations of the humans, VAA 112 causes displayed GUI 400 to be updated with the real-time locations of the humans.

Furthermore, each location identifier, of the one or more location identifiers, is visually associated with one or more displayed health-interaction values, of the health-interaction data, for a human associated with said each location identifier. As the location indicators move around map 410, the associated health-interaction values displayed in the GUI remain in visual association with the location indicators (i.e., the health-interaction values move around the map with the location indicators).

According to an embodiment, VAA 112 receives a real-time update for one or more health-interaction values displayed on the map GUI. For example, one of the humans leaves the monitored physical area (e.g., for lunch or an off-site meeting), and interacts with a health kiosk of health kiosk system 150 upon returning to the monitored physical area. During the interaction, the health kiosk records an updated body temperature for the human. Health kiosk system 150 sends a real-time update of the data to VAA 112, which updates the displayed health-interaction value associated with the location indicator for the human.

### i. Health Warning Criteria

According to an embodiment, VAA 112 maintains health warning criteria. For example, VAA 112 determines that a particular human satisfies health warning criteria based on one or more of: a body temperature of the particular human surpassing a temperature threshold; an illness diagnosis of the particular human; a reported red-flag symptom for the particular human; a plurality of previous distancing criteria violations involving the particular human; or a previous distancing criteria violation involving both (a) the particular human, and (b) another human with an illness diagnosis that occurred within a threshold amount of time of a timestamp associated with the previous distancing criteria violation. According to an embodiment, upon determining that a particular human satisfies maintained health warning criteria, VAA 112 includes, in the graphical user interface, a visual highlight for the location identifier of the particular human to indicate that the human is associated with a health warning.

For example, VAA 112 maintains health warning criteria of a body temperature reading that is below the threshold body temperature for entering the monitored physical area (e.g., 100.4°F), but is above a lower warning threshold body temperature (e.g., 99.0°F). Person C (associated with location indicator 412C of GUI 400) interacts with a health kiosk of system 150, during which the health kiosk measures a body temperature of 99.2°F for Person C. Based on determining that this body temperature value satisfies the health warning criteria, VAA 112 associates the location indicator of the human with a health warning visual highlight, such as icon 416. The visual highlight may be any kind of visual highlight associated with the location indicator, including a background color, a text color, a text weight, a font, underlining, italicizing, animation of the location indicator (such as blinking), any style of icon placed anywhere in the vicinity of the health-interaction data or the location indicator value, a color of the associated location indicator, a shape of the associated location indicator, etc.

### ii. Health Status Indicator

According to an embodiment, the health-interaction information displayed on the map of the monitored physical area comprises a health status indicator that is determined from a plurality of health-interaction values, of the health-interaction data, for the associated human. Because the health status indicator is calculated from multiple health-interaction values, display of this indicator provides a way for a user of the GUI to quickly make decisions based on multiple factors at once, i.e., the factors that go into calculating the health status indicator.

According to an embodiment, VAA 112 maintains administrator parameter information that indicates a plurality of factors to use in calculating health status indicators for the humans in the monitored physical area. For example, based on the administrator parameter information, VAA 112 determines a health status indicator for each human based on whether the human satisfies health warning criteria and also based on answers to one or more survey questions gathered by the health kiosk (such as "Do you have any of the following symptoms?" and "Have you had close contact with anyone with the following symptoms?"). To illustrate, each of these factors of the health status indicator has a "desirable" and an "undesirable" value. If all of the factors have undesirable values, then the health status indicator is at a HIGH warning level. If only two factors have undesirable values, then the health status indicator is at a MEDIUM warning level. If only one factor has an undesirable value, then the health status indicator is at a LOW warning level. If all factors have desirable values, then the health status indicator is at a NO WARNING level. While this illustrative calculation of the health status indicator is based on boolean values, calculation of a health status indicator may also be based on numerical values.

For example, Person A that is associated with location indicator 412A has a body temperature that does not satisfy the health warning criteria (a desirable value), and answered NO to "Do you have any of the following symptoms?" (a desirable value), but answered YES to "Have you had close contact with anyone with the following symptoms?" (an undesirable value). Based on there being only one factor with an undesirable value, the health status indicator for Person A is at a LOW warning level.

As another example, Person B that is associated with location indicator 412B has a body temperature that does not satisfy the health warning criteria (a desirable value), and answered NO to both "Do you have any of the following symptoms?" and "Have you had close contact with anyone with the following symptoms?" (desirable values). Based on having only factors with desirable values, the health status indicator for Person B is at a NO WARNING level.

The health status indicator generated for a human may be displayed in visual association with the location identifier of the human in any way, including as a label, as a symbol, as a color of the location indicator of the human, etc. For example, the location indicator for a human associated with the following health status indicator levels have the associated color: a NO WARNING level is green, a LOW warning level is yellow, a MEDIUM warning level is orange, and a HIGH warning level is red.

### B. Graphical User Interface with Elevated-Risk Distancing Criteria Violation Data

According to an embodiment, VAA 112 generates graphical user interface data that includes one or more alerts of enhanced-risk distancing criteria violations in a monitored physical area. An enhanced-risk distancing criteria violation is an instance of distancing criteria violation between two or more humans in the monitored physical area, where one or more of the humans is also associated with a health warning.

For example, VAA 112 receives data, from location system 140, that comprises location information for a plurality of humans in the monitored physical area. VAA 112 also receives health-interaction data, from health kiosk system 150, that comprises one or more health-interaction values for each human of the plurality of humans. VAA 112 determines that the location information, for two or more humans of the plurality of humans, indicates a violation of distancing criteria. For example, the distancing criteria applied by VAA 112 comprises a minimum distance to be maintained between humans (e.g., six feet) and a maximum amount of time that the minimum distance may be breached before the distancing criteria is violated (e.g., 15 minutes). VAA 112 further determines that the distance criteria violation is an enhanced-risk distancing criteria violation based on determining that one or more humans, of the two or more humans, satisfy health warning criteria. Accordingly, VAA 112 causes an alert of the enhanced-risk distancing criteria violation to be included in the graphical user interface.

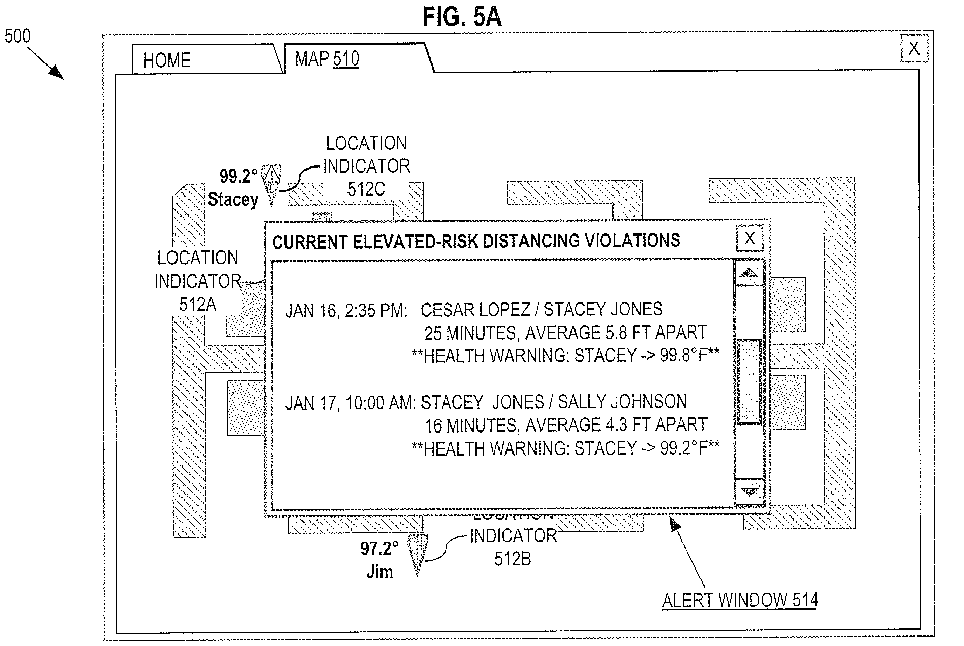

For example, FIG. 5A depicts an example GUI 500 with an alert window 514 that includes a list of elevated-risk distancing criteria violations that have occurred within the monitored physical area. Example alert window 514 is displayed as being superimposed over a map 510 that includes location indicators 512A-C, according to the embodiment described in connection with FIG. 4 (i.e., displaying health-interaction values with real time location indicators). However, embodiments are not limited to being displayed with a map.

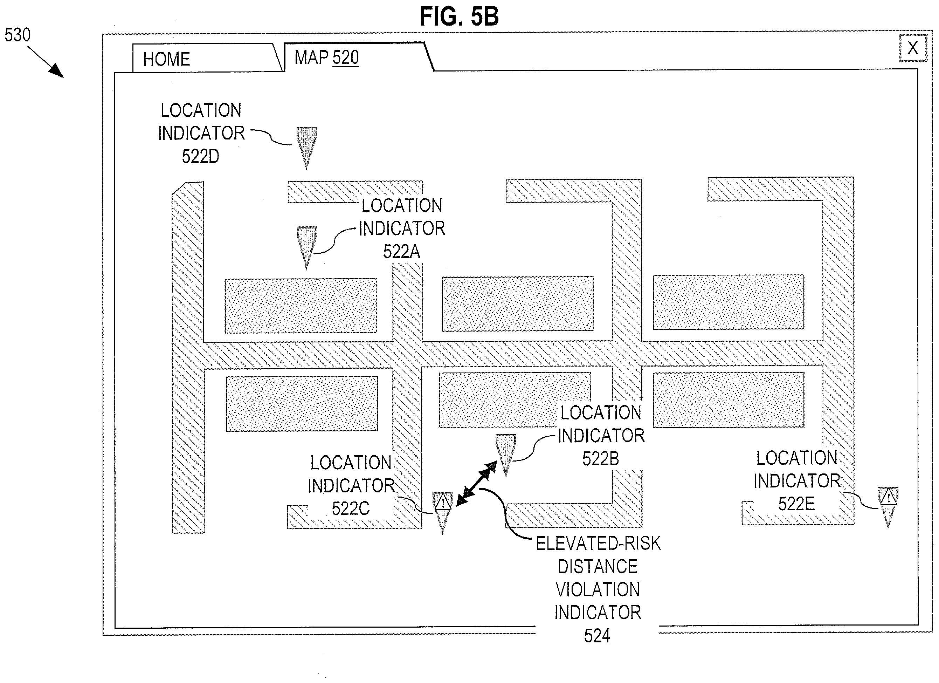

According to an embodiment, VAA 112 causes display of an elevated-risk distancing criteria violation on a map depiction of the monitored physical area. To illustrate, the data received by VAA 112 includes location data, e.g., as part of the location data from location system 140. Based on the location data, VAA 112 populates the map depiction, of the graphical user interface, with a plurality of location identifiers of the plurality of humans. For example, FIG. 5B depicts an example GUI 530 that includes a map 520 of the monitored physical area, which displays alerts for distancing criteria violations involving elevated risk. In example GUI 530, map 520 includes five location indicators 522A-E that show the locations for five respective humans in the monitored physical area.

VAA 112 visually associates a visual indication of the enhanced-risk distancing criteria violation with the location identifiers of the two or more humans involved in the violation. To illustrate in the context of GUI 530, Person A associated with location indicator 522A and Person D associated with location indicator 522D are within six feet of each other. However, Person D is merely passing the cubicle of Person A, and is not within six feet of Person A for more than the maximum 15 minutes. In contrast, Person C associated with location indicator 522C and Person B associated with location indicator 522B have been within six feet of each other for more than the threshold 15 minutes (chatting at the cubicle of Person B). Person C is associated with a health warning because the health-interaction data of Person C satisfies health warning criteria (e.g., Person C has a body temperature of over 99.0°F). Thus, VAA 112 determines that Person B and Person C are involved in an elevated-risk distancing criteria violation.

As shown in map 520, based on the detection of the elevated-risk distancing criteria violation between Person B and Person C, VAA 112 causes the graphical user interface to include elevated-risk distancing criteria violation indicator 524 in visual association with location indicator 522B and location indicator 522C.

According to an embodiment, as VAA 112 receives real-time updates for locations of the humans, VAA 112 causes GUI 530 to be updated with the real-time locations of the humans. As the location indicators move around map 520, VAA 112 monitors the location information to identify distancing criteria violations, as indicated above. According to an embodiment, as the humans involved in an elevated-risk distancing criteria violation move, thereby satisfying distancing criteria, the alert for the violation is removed from the GUI.

### C. Contact Tracing

According to an embodiment, an elevated-risk distancing criteria violation between multiple humans that involves a health warning based on previous distancing criteria violation is referred to herein as a "contact-tracing" distancing violation. According to an embodiment, a health warning based on a previous distancing criteria violation is a previous distancing criteria violation involving both (a) a particular human from the current distancing violation, and (b) another human with an illness diagnosis that occurred within a threshold amount of time of a timestamp associated with the previous distancing criteria violation.

According to an embodiment, a contact-tracing distancing violation is identified in the GUI using a particular contact tracing alert marking. For example, a contact-tracing distancing violation may be marked with a "contact tracing" symbol, e.g., on the location marker of the individual that came into close contact with another human with an illness diagnosis.

According to an embodiment, VAA 112 maintains historical distancing criteria violations data within a workplace health data repository. For example, VAA 112 records distancing criteria violations with details of any associated health warnings. As another example, VAA 112 retroactively marks distancing violations as elevated-risk when relevant illness diagnoses are made known to the system. For example, an illness diagnosis may be reported to administrators of the monitored physical area (e.g., Human Resources for the company, or a supervisor of the person that was diagnosed), may be self-reported via a health kiosk of health kiosk system 150, etc.

In addition to providing a visual indication on the graphical user of health status, health warnings, distance criteria violations, etc., the VAA 112 may generate and transmit one or more messages to specify this information. For example, the VAA 112 generates and transmits one or more messages to an administrator user, health administrator, security personnel, etc., to specify the health status, health warnings, distance criteria violations, etc., to provide remote notification.

### D. Graphical User Interface with Composite Statistics

According to an embodiment, VAA 112 generates graphical user interface data that comprises composite statistics with information from location system 140 and health kiosk system 150. For example, VAA 112 generates GUI data that includes a chart or graph (such as a line graph, a pie chart, a bar graph, a mosaic chart, a heat map, etc.) that represents a percentage of people not wearing masks that are located within a portion or all of a monitored physical area (such as in the rooms of a monitored physical area, the floors of a monitored physical area, etc.), that represents a number of enhanced-risk distancing violations per person, statistics of recorded body temperatures for people within a portion or all of a monitored physical area, etc.

For example, VAA 112 receives data, from location system 140, that comprises location information for a plurality of humans in the monitored physical area. VAA 112 also receives health-interaction data, from health kiosk system 150, that comprises one or more health-interaction values for each human of the plurality of humans. VAA 112 automatically generates one or more charts or graphs to represent composite statistics in the information received from location system 140 and health kiosk system 150.

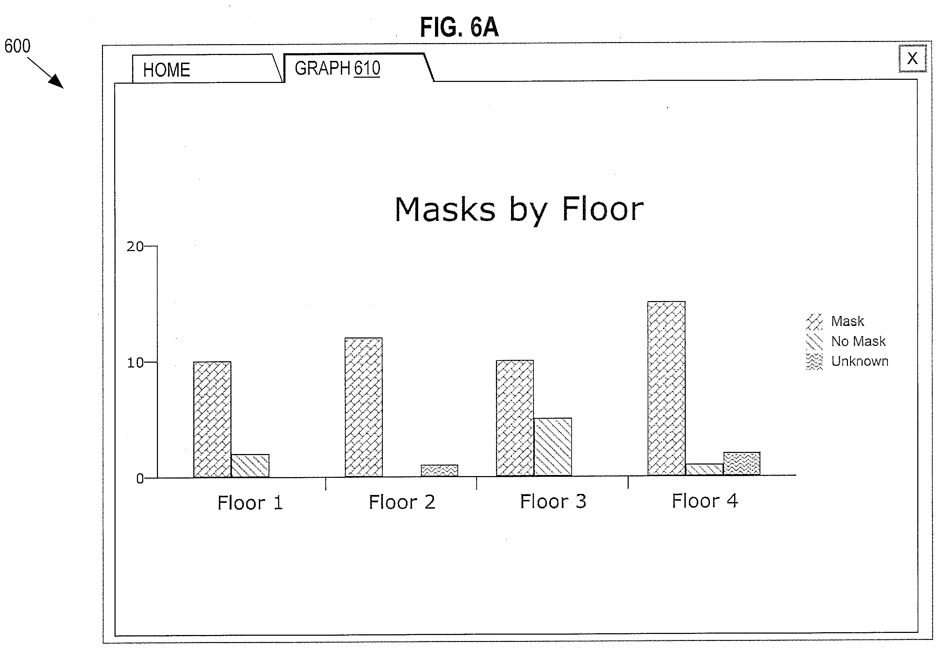

In an example, a monitored work area has four floors with 12-18 people occupying each floor. The location information from location system 140 includes locations for people within the monitored physical area, including which floor each person currently occupies. The health-interaction data from health kiosk system 150 includes whether a mask was detected on each person's face, where the result may be "unknown" in cases where the computer vision application was not able to make a mask determination (e.g., the person's face was out of range of the camera sensor during the health kiosk interaction). FIG. 6A depicts a GUI 600 with a bar graph 610 that indicates the number of people on each floor that are wearing masks, not wearing masks, and "unknown".

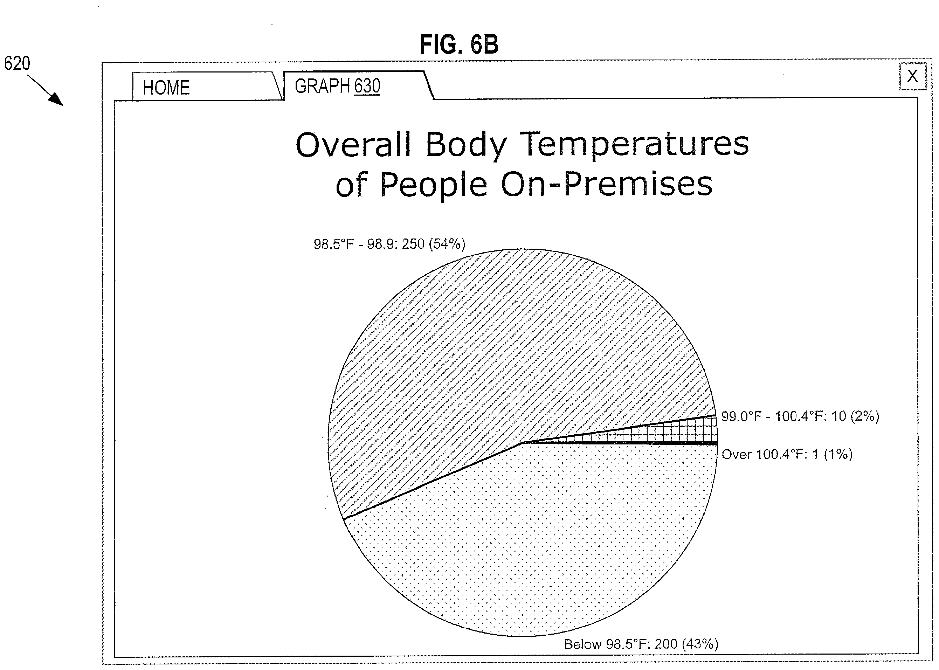

In another example, the location information from location system 140 includes locations for people within the monitored physical area, including which people are within the monitored physical area (i.e., "on premises") at any given time. The health-interaction data from health kiosk system 150 includes the detected body temperature of each person within the monitored physical area. FIG. 6B depicts a GUI 620 with a pie chart 630 that indicates the percentages of people, currently on-premises, that have temperatures below 98.5°F, between 98.5°F and 98.9°F, between 99.0°F and 100.4°F, and over 100.4°F (e.g., a person in upper management who is able to override the requirement to be under 100.4°F in order to enter the monitored physical area).

### IV. Workplace Health Data Repository

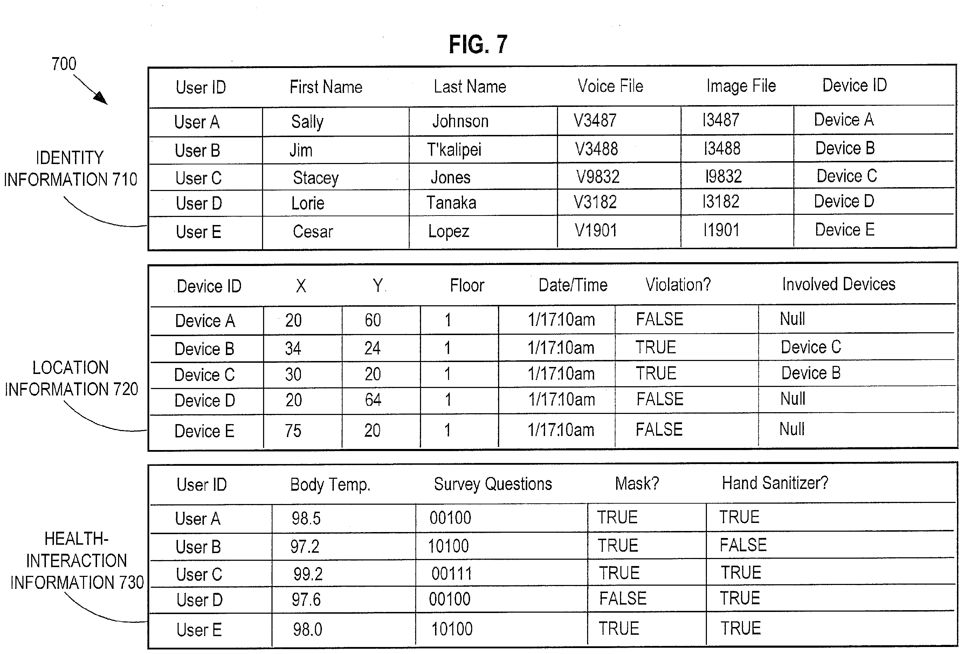

FIG. 7 depicts contents of an example workplace health data repository 700 maintained by VAA 112. Specifically, example repository 700 includes the following example database objects, e.g., tables where each row corresponds to a particular person (employee or visitor): identity information 710, location information 720, and health-interaction information 730.

Although identity information 710, location information 720, and health-interaction information 730 are depicted as having five entries each, embodiments are not limited to this example and may include any amount of data. In addition, identity information 710, location information 720, and health-interaction information 730 may be sorted and/or indexed within repository 700 in various ways that may vary depending upon a particular implementation.

### A. Identity Information

Identity information 710 may be created and maintained in several different ways that may vary depending upon a particular implementation. For example, identity information may be manually created and maintained by an administrator of an organization, such as human resource personnel, during the hiring process or new employee orientation.

In example repository 700, identity information 710 includes a user ID, a first name, a last name, voice data, image data, and a device ID. Identity information 710 is not limited to the example information in FIG. 7, and may include any type of information about a person, such as occupation, educational background, work history, accomplishments and achievements, personal interests, physical attributes, such as eye color, hair color, skin color, distinguishing marks, weight, size, etc.

The user ID may be any data (such as an alphanumeric string) that uniquely identifies a person, such as an employee identifier, a visitor identifier, etc.

Voice data may be any type of audio data that corresponds to a person, and is represented in example repository 700 as a reference to a voice file. The audio data may be used to help identify the person. For example, voice data may represent one or more words spoken by an individual, such as their name or a particular phrase. Voice data may be in any format that may vary depending upon a particular implementation, and embodiments are not limited to any particular format of voice data. Example formats for voice data include, without limitation, WMA, WAV, Real Audio (.ra, .ram, .rm), MIDI, Ogg, etc. Voice data may be used alone, or in combination with the image data, to identify a speaker.

Image data may be any type of image data that corresponds to the person, which may be used to help identify the person. Image data is represented in example repository 700 as a reference to an image file. For example, the image file may include one or more facial images. Image data may also include other data that represents a result of processing one or more images. This type of information may be helpful when attempting to match one or more collected or received images with the image data from identity information 710. For example, image data may include an image signature, the result of processing one or more images with one or more algorithms, hash functions, etc. Image data may be in any format that may vary depending upon a particular implementation, and embodiments are not limited to any particular format of image data. Example formats for image data include, without limitation, JPG, TIF, GIF, PNG, RAW, etc.

The device ID may be any data that uniquely identifies a device that is being used by the person, such as the serial number, MAC address, or other identifying information of a device carried by the person, an identifier of a chip carried by the person, etc. According to an embodiment, the identified device is a location-aware device or identifier device that is used to determine the person's location by location system 140.

### B. Location Information

According to an embodiment, VAA 112 retrieves, from location system 140 via an API, location information 720 for humans in the monitored physical area. In example repository 700, location information 720 includes a device ID, an X location component, a Y location component, a floor location component, a date/timestamp, a boolean indication of whether the user is currently involved in a distancing criteria violation, and an indication of other device IDs involved in the violation (if applicable). Location information 720 is not limited to the example information in FIG. 7, and may include any type of information relevant to a person's location, including a user ID of the person.

The device ID may be any data that uniquely identifies a device that is being used to track a person's location, such as the serial number, MAC address, or other identifying information of a device carried by the person. According to an embodiment, the device ID of location information 720 corresponds to the device ID of identity information 710.

The X, Y, and floor (or Z) location components in location information 720 identify the exact location of the person within the monitored physical area. The date/timestamp indicates the latest timestamp at which the data was updated. The violation information indicates whether location system 140 has detected a violation of maintained distancing criteria, and the involved devices column indicates any device identifier that is also involved in any indicated distancing criteria violation.

### C. Health-Interaction Information

According to one embodiment, VAA 112 retrieves, from health kiosk system 150 via an API, health-interaction information 730 for humans in the monitored physical area. In example repository 700, health-interaction information 730 includes a user ID, a body temperature value, boolean answers to five survey questions, a boolean value indicating whether a mask was detected, and a boolean value indicating whether hand sanitizer was dispensed to the person.

The user ID is data that uniquely identifies the person, and corresponds to the user ID in identity information 710. For example, the user ID is read at a health kiosk by scanning a user identification card presented to the health kiosk. The body temperature value is detected by a health kiosk, e.g., using automatic touchless temperature technology. The health kiosk gathers answers to survey questions, e.g., by using computer vision to detect whether the user shakes or nods their head in response to presentation of each survey question at a display device of the kiosk, by detecting audible answers using computer voice recognition, etc. Detection of a mask may be performed by a health kiosk by taking a picture of the user and using computer vision to detect the presence or absence of a mask. Furthermore, touchless technology is used to dispense hand sanitizer to a user, which is recorded by the health kiosk.

### V. Architecture

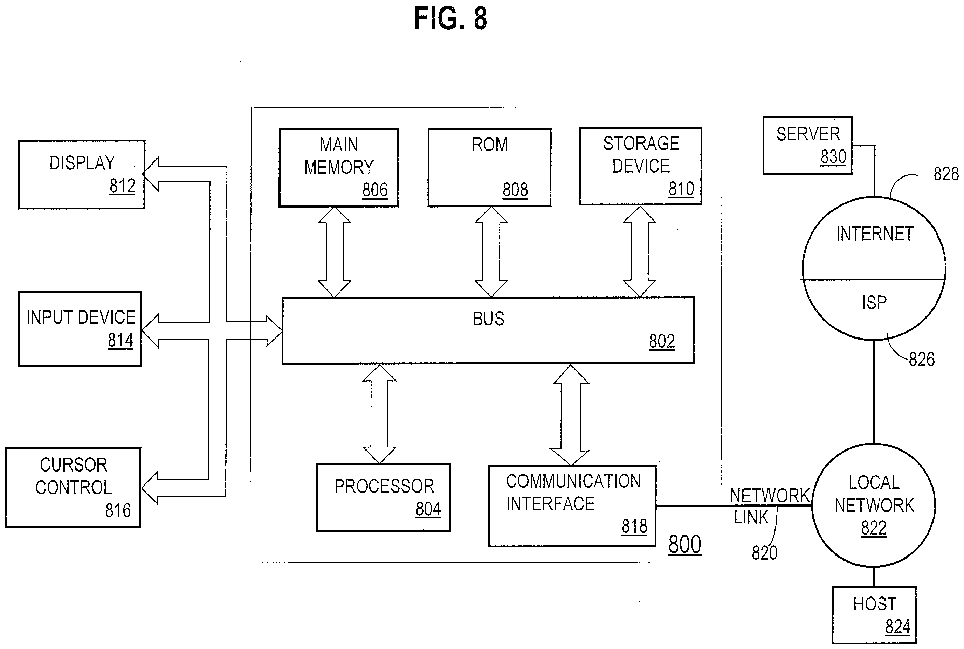

In an embodiment, arrangement 100 of FIG. 1 involves computers, i.e., computing device 110, client device 130, location system 140, and health kiosk system 150. A "computer" may be one or more physical computers, virtual computers, and/or computing devices. A computer may be a client and/or a server. Any reference to "a computer" herein may mean one or more computers, unless expressly stated otherwise. Each of the logical and/or functional units depicted in any of the figures or described herein may be implemented using any of the techniques further described herein in connection with FIG. 8. The one or more computing devices may be special-purpose computing devices, or generic computing devices, and may be implemented using any combination of computer hardware, computer software, and/or computer firmware. VAA 112 may be replicated over multiple computing devices such that at any point in time, at least one computing device can provide meeting intelligence services.

For purposes of explanation, VAA 112 is depicted as running on a computing device 110. However, one or more applications implementing the functionality attributed herein to VAA 112, location system 140, health kiosk system 150, or other information source systems described herein, could be implemented on one or more computing devices or could be implemented as one or more cloud-based applications.

Network 120 may be implemented by one or more wired or wireless networks, such as one or more local area networks (LANs), wide area networks (WANs), the Internet, etc. The elements in arrangement 100 may also have direct connections and embodiments are not limited to the example elements depicted in arrangement 100 and fewer or additional elements may be used, depending upon a particular implementation.

Client device 130 may be any type of device that is capable of displaying a GUI as described in detail herein. Examples of client device 130 include, without limitation, desktop computers, notebook computers, tablet computing devices, mobile communications devices such as smartphones, smart watches, etc. Client device 130 may include various processes used to support display of GUIs. For example, client device 130 may include a workplace health safety client that displays the GUI to a user of client device 130. As another example, client device 130 may include a web browser that displays the GUI.

In an embodiment, VAA 112 is communicatively coupled to a workplace health data repository 700 (not depicted in FIG. 1). The workplace health data repository 700 may be implemented as a database, data structure, a configuration file, and/or any other system that stores data gathered or generated by VAA 112.

### VI. Implementation Examples

Although the flow diagrams of the present application depict a particular set of steps in a particular order, other implementations may use fewer or more steps, in the same or different order, than those depicted in the figures.

According to one embodiment, the techniques described herein are implemented by one or more special-purpose computing devices. The special-purpose computing devices may be hard-wired to perform the techniques, or may include digital electronic devices such as one or more application-specific integrated circuits (ASICs) or field programmable gate arrays (FPGAs) that are persistently programmed to perform the techniques, or may include one or more general purpose hardware processors programmed to perform the techniques pursuant to program instructions in firmware, memory, other storage, or a combination. Such special-purpose computing devices may also combine custom hard-wired logic, ASICs, or FPGAs with custom programming to accomplish the techniques. The special-purpose computing devices may be desktop computer systems, portable computer systems, handheld devices, networking devices or any other device that incorporates hard-wired and/or program logic to implement the techniques.

For example, FIG. 8 is a block diagram that illustrates a computer system 800 upon which an embodiment of the invention may be implemented. Computer system 800 includes a bus 802 or other communication mechanism for communicating information, and a hardware processor 804 coupled with bus 802 for processing information. Hardware processor 804 may be, for example, a general purpose microprocessor.

Computer system 800 also includes a main memory 806, such as a random access memory (RAM) or other dynamic storage device, coupled to bus 802 for storing information and instructions to be executed by processor 804. Main memory 806 also may be used for storing temporary variables or other intermediate information during execution of instructions to be executed by processor 804. Such instructions, when stored in non-transitory storage media accessible to processor 804, render computer system 800 into a special-purpose machine that is customized to perform the operations specified in the instructions.

Computer system 800 further includes a read only memory (ROM) 808 or other static storage device coupled to bus 802 for storing static information and instructions for processor 804. A storage device 810, such as a magnetic disk, optical disk, or solid-state drive is provided and coupled to bus 802 for storing information and instructions.

Computer system 800 may be coupled via bus 802 to a display 812, such as a cathode ray tube (CRT), for displaying information to a computer user. An input device 814, including alphanumeric and other keys, is coupled to bus 802 for communicating information and command selections to processor 804. Another type of user input device is cursor control 816, such as a mouse, a trackball, or cursor direction keys for communicating direction information and command selections to processor 804 and for controlling cursor movement on display 812. This input device typically has two degrees of freedom in two axes, a first axis (e.g., x) and a second axis (e.g., y), that allows the device to specify positions in a plane.

Computer system 800 may implement the techniques described herein using customized hard-wired logic, one or more ASICs or FPGAs, firmware and/or program logic which in combination with the computer system causes or programs computer system 800 to be a special-purpose machine. According to one embodiment, the techniques herein are performed by computer system 800 in response to processor 804 executing one or more sequences of one or more instructions contained in main memory 806. Such instructions may be read into main memory 806 from another storage medium, such as storage device 810. Execution of the sequences of instructions contained in main memory 806 causes processor 804 to perform the process steps described herein. In alternative embodiments, hard-wired circuitry may be used in place of or in combination with software instructions.

The term "storage media" as used herein refers to any non-transitory media that store data and/or instructions that cause a machine to operate in a specific fashion. Such storage media may comprise non-volatile media and/or volatile media. Non-volatile media includes, for example, optical disks, magnetic disks, or solid-state drives, such as storage device 810. Volatile media includes dynamic memory, such as main memory 806. Common forms of storage media include, for example, a floppy disk, a flexible disk, hard disk, solid-state drive, magnetic tape, or any other magnetic data storage medium, a CD-ROM, any other optical data storage medium, any physical medium with patterns of holes, a RAM, a PROM, and EPROM, a FLASH-EPROM, NVRAM, any other memory chip or cartridge.

Storage media is distinct from but may be used in conjunction with transmission media. Transmission media participates in transferring information between storage media. For example, transmission media includes coaxial cables, copper wire and fiber optics, including the wires that comprise bus 802. Transmission media can also take the form of acoustic or light waves, such as those generated during radio-wave and infra-red data communications.

Various forms of media may be involved in carrying one or more sequences of one or more instructions to processor 804 for execution. For example, the instructions may initially be carried on a magnetic disk or solid-state drive of a remote computer. The remote computer can load the instructions into its dynamic memory and send the instructions over a telephone line using a modem. A modem local to computer system 800 can receive the data on the telephone line and use an infra-red transmitter to convert the data to an infra-red signal. An infra-red detector can receive the data carried in the infra-red signal and appropriate circuitry can place the data on bus 802. Bus 802 carries the data to main memory 806, from which processor 804 retrieves and executes the instructions. The instructions received by main memory 806 may optionally be stored on storage device 810 either before or after execution by processor 804.

Computer system 800 also includes a communication interface 818 coupled to bus 802. Communication interface 818 provides a two-way data communication coupling to a network link 820 that is connected to a local network 822. For example, communication interface 818 may be integrated services digital network (ISDN) card, cable modem, satellite modem, or a modem to provide a data communication connection to a corresponding type of telephone line. As another example, communication interface 818 may be a local area network (LAN) card to provide a data communication connection to a compatible LAN. Wireless links may also be implemented. In any such implementation, communication interface 818 sends and receives electrical, electromagnetic or optical signals that carry digital data streams representing various types of information.

Network link 820 typically provides data communication through one or more networks to other data devices. For example, network link 820 may provide a connection through local network 822 to a host computer 824 or to data equipment operated by an Internet Service Provider (ISP) 826. ISP 826 in turn provides data communication services through the world wide packet data communication network now commonly referred to as the "Internet" 828. Local network 822 and Internet 828 both use electrical, electromagnetic or optical signals that carry digital data streams. The signals through the various networks and the signals on network link 820 and through communication interface 818, which carry the digital data to and from computer system 800, are example forms of transmission media.

Computer system 800 can send messages and receive data, including program code, through the network(s), network link 820 and communication interface 818. In the Internet example, a server 830 might transmit a requested code for an application program through Internet 828, ISP 826, local network 822 and communication interface 818.

The received code may be executed by processor 804 as it is received, and/or stored in storage device 810, or other non-volatile storage for later execution.

### CLOUD COMPUTING

The term "cloud computing" is generally used herein to describe a computing model which enables on-demand access to a shared pool of computing resources, such as computer networks, servers, software applications, and services, and which allows for rapid provisioning and release of resources with minimal management effort or service provider interaction.

A cloud computing environment (sometimes referred to as a cloud environment, or a cloud) can be implemented in a variety of different ways to best suit different requirements. For example, in a public cloud environment, the underlying computing infrastructure is owned by an organization that makes its cloud services available to other organizations or to the general public. In contrast, a private cloud environment is generally intended solely for use by, or within, a single organization. A community cloud is intended to be shared by several organizations within a community; while a hybrid cloud comprises two or more types of cloud (e.g., private, community, or public) that are bound together by data and application portability.

Generally, a cloud computing model enables some of those responsibilities which previously may have been provided by an organization's own information technology department, to instead be delivered as service layers within a cloud environment, for use by consumers (either within or external to the organization, according to the cloud's public/private nature). Depending on the particular implementation, the precise definition of components or features provided by or within each cloud service layer can vary, but common examples include: Software as a Service *(SaaS),* in which consumers use software applications that are running upon a cloud infrastructure, while a *SaaS* provider manages or controls the underlying cloud infrastructure and applications. **Platform as a Service** (PaaS), in which consumers can use software programming languages and development tools supported by a PaaS provider to develop, deploy, and otherwise control their own applications, while the PaaS provider manages or controls other aspects of the cloud environment (i.e., everything below the run-time execution environment). **Infrastructure as a Service** (IaaS), in which consumers can deploy and run arbitrary software applications, and/or provision processing, storage, networks, and other fundamental computing resources, while an IaaS provider manages or controls the underlying physical cloud infrastructure (i.e., everything below the operating system layer). **Database as a Service** (*DBaaS*) in which consumers use a database server or Database Management System that is running upon a cloud infrastructure, while a *DbaaS* provider manages or controls the underlying cloud infrastructure, applications, and servers, including one or more database servers.

In the foregoing specification, embodiments of the invention have been described with reference to numerous specific details that may vary from implementation to implementation. The specification and drawings are, accordingly, to be regarded in an illustrative rather than a restrictive sense. The sole and exclusive indicator of the scope of the invention, and what is intended by the applicants to be the scope of the invention, is the literal and equivalent scope of the set of claims that issue from this application, in the specific form in which such claims issue, including any subsequent correction.

This application is based on U.S. Patent Application No. 17/198,136, filed March 10, 2021, the content of which is incorporated herein by reference.

### EXAMPLES

[Example 1] One or more non-transitory computer readable media storing one or more sequences of instructions that, when processed by one or more processors, cause:
receiving data from a set of information sources including a location information system (140) and a health kiosk information system (150);
said receiving data including, for each information source of the set of information sources, receiving data via an application programming interface (API) for said each information source;
generating graphical user interface data which, when processed at a computing device (110), causes a graphical user interface to be displayed that visually aggregates representations of the received data from the set of information sources; and
causing the graphical user interface data to be transmitted to a client computing device (130).

[Example 2] The one or more non-transitory computer readable media of example 1,
the received data including:
   location data including one or more locations for one or more respective humans within a monitored physical area, and
   health-interaction data including one or more health-interaction values for each human of the one or more humans;
the graphical user interface including:
   a map depiction of the monitored physical area with one or more location identifiers of the one or more humans, locations, on the map depiction of the monitored physical area, of the one or more location identifiers being determined based on the location data, and
   each location identifier, of the one or more location identifiers, being visually associated with one or more displayed health-interaction values, of the health-interaction data, for a human associated with said each location identifier.

[Example 3] The one or more non-transitory computer readable media of example 2,
the location data being real-time location data;
the one or more real-time locations for the one or more respective humans being real-time locations; and
the one or more location identifiers in the map depiction of the monitored physical area reflecting the real-time locations for the one or more humans.

[Example 4] The one or more non-transitory computer readable media of example 2 or 3, the health-interaction data being received from the health kiosk information system and the location data being received from the location information system.

[Example 5] The one or more non-transitory computer readable media of any one of examples 2 to 4, the one or more sequences of instructions further including instructions that, when processed by one or more processors, cause:
for each human of the one or more humans, determining a health status indicator from a plurality of health-interaction values, of the health-interaction data, for said each human;
said one or more displayed health-interaction values visually associated with each location identifier of the one or more location identifiers including the health status indicator determined for the human associated with said each location identifier.

[Example 6] The one or more non-transitory computer readable media of any one of examples 1 to 5,
the received data including:
   location information for a plurality of humans in a monitored physical area, and
   health-interaction data including one or more health-interaction values of each human of the plurality of humans; and
the one or more sequences of instructions further including instructions that, when processed by one or more processors, cause:
   determining that the location information, for two or more humans of the plurality of humans, indicates a violation of distancing criteria;
   determining that the distancing criteria violation is an enhanced-risk distancing criteria violation based on determining that one or more humans, of the two or more humans, satisfy health warning criteria; and
   the graphical user interface including an alert of the enhanced-risk distancing criteria violation.

[Example 7] The one or more non-transitory computer readable media of example 6,
said determining that the distancing criteria violation is an enhanced-risk distancing criteria violation being based on determining that a particular human, of the two or more humans, satisfies health warning criteria based on a previous distancing criteria violation involving both (a) the particular human, and (b) another human with an illness diagnosis that occurred within a threshold amount of time of a timestamp associated with the previous distancing criteria violation;
the graphical user interface including a contact tracing alert marking that is visually associated with the alert of the enhanced-risk distancing criteria violation.

[Example 8] The one or more non-transitory computer readable media of example 6 or 7,
the received data including location data; and
the graphical user interface further including:
   a map depiction of the monitored physical area with a plurality of location identifiers of the plurality of humans,
   locations, on the map depiction of the monitored physical area, of the plurality of location identifiers being determined based on the location data, and
   the alert including a visual indication of the enhanced-risk distancing criteria violation that is visually associated with the location identifiers of the two or more humans.

### REFERENCE SIGNS LIST

- 110: Computing device
- 130: Client device
- 140: Location system
- 150: Health kiosk system
- 800: Computing system
- 804: Processors
- 806: Main memory
- 808: ROM
- 810: Storage device

## Claims

1. A computing system (800) comprising:
one or more processors (804); and
one or more memories (806,808,810) storing one or more sequences of instructions that, when processed by the one or more processors, cause:
receiving data from a set of information sources comprising a location information system and a health kiosk information system;
wherein said receiving data comprises, for each information source of the set of information sources, receiving data via an application programming interface (API) for said each information source;
generating graphical user interface data which, when processed at a computing device (110), causes a graphical user interface to be displayed that visually aggregates representations of the received data from the set of information sources; and
causing the graphical user interface data to be transmitted to a client computing device (130).

2. The computing system of Claim 1, wherein:
the received data comprises:
location data comprising one or more locations for one or more respective humans within a monitored physical area, and
health-interaction data comprising one or more health-interaction values for each human of the one or more humans;
the graphical user interface comprises:
a map depiction of the monitored physical area with one or more location identifiers of the one or more humans, wherein locations, on the map depiction of the monitored physical area, of the one or more location identifiers are determined based on the location data, and
wherein each location identifier, of the one or more location identifiers, is visually associated with one or more displayed health-interaction values, of the health-interaction data, for a human associated with said each location identifier.

3. The computing system of Claim 2, wherein:
the location data is real-time location data;
the one or more real-time locations for the one or more respective humans are real-time locations; and
the one or more location identifiers in the map depiction of the monitored physical area reflect the real-time locations for the one or more humans.

4. The computing system of Claim 2 or 3, wherein the health-interaction data is received from the health kiosk information system (150) and the location data is received from the location information system (140).

5. The computing system of any one of Claims 2 to 4, wherein the one or more sequences of instructions further comprise instructions that, when processed by the one or more processors, cause:
for each human of the one or more humans, determining a health status indicator from a plurality of health-interaction values, of the health-interaction data, for said each human;
wherein said one or more displayed health-interaction values visually associated with each location identifier of the one or more location identifiers comprises the health status indicator determined for the human associated with said each location identifier.

6. The computing system of any one of Claims 1 to 5, wherein:
the received data comprises:
location information for a plurality of humans in a monitored physical area, and
health-interaction data comprising one or more health-interaction values of each human of the plurality of humans; and
the one or more sequences of instructions further comprise instructions that, when processed by the one or more processors, cause:
determining that the location information, for two or more humans of the plurality of humans, indicates a violation of distancing criteria;
determining that the distancing criteria violation is an enhanced-risk distancing criteria violation based on determining that one or more humans, of the two or more humans, satisfy health warning criteria; and
wherein the graphical user interface comprises an alert of the enhanced-risk distancing criteria violation.

7. The computing system of Claim 6, wherein the location information is real-time location information.

8. The computing system of Claim 6 or 7, wherein:
said determining that the distancing criteria violation is an enhanced-risk distancing criteria violation is based on determining that a particular human, of the two or more humans, satisfies health warning criteria based on a previous distancing criteria violation involving both (a) the particular human, and (b) another human with an illness diagnosis that occurred within a threshold amount of time of a timestamp associated with the previous distancing criteria violation;
the graphical user interface comprises a contact tracing alert marking that is visually associated with the alert of the enhanced-risk distancing criteria violation.

9. The computing system of any one of Claims 6 to 8, wherein:
the graphical user interface further comprises:
a map depiction of the monitored physical area with a plurality of location identifiers of the plurality of humans, wherein locations, on the map depiction of the monitored physical area, of the plurality of location identifiers are determined based on the location data, and
wherein the alert comprises a visual indication of the enhanced-risk distancing criteria violation that is visually associated with the location identifiers of the two or more humans.

10. The computing system of any one of Claims 6 to 9, wherein the distancing criteria comprises a minimum distance to be maintained between humans and a maximum amount of time that the minimum distance may be breached before the distancing criteria is violated.

11. The computing system of any one of Claims 6 to 10, wherein determining that a particular human, of the one or more humans, satisfies the health warning criteria is based on one or more of:
a body temperature of the particular human surpassing a temperature threshold;
an illness diagnosis of the particular human;
a reported red-flag symptom for the particular human;
a plurality of previous distancing criteria violations involving the particular human; or
a previous distancing criteria violation involving both (a) the particular human, and (b) another human with an illness diagnosis that occurred within a threshold amount of time of a timestamp associated with the previous distancing criteria violation.

12. One or more non-transitory computer readable media storing one or more sequences of instructions that, when processed by one or more processors, cause:
receiving data from a set of information sources comprising a location information system (140) and a health kiosk information system (150);
wherein said receiving data comprises, for each information source of the set of information sources, receiving data via an application programming interface (API) for said each information source;
generating graphical user interface data which, when processed at a computing device (110), causes a graphical user interface to be displayed that visually aggregates representations of the received data from the set of information sources; and
causing the graphical user interface data to be transmitted to a client computing device (130).

13. The one or more non-transitory computer readable media of Claim 12, wherein:
the received data comprises:
location data comprising one or more locations for one or more respective humans within a monitored physical area, and
health-interaction data comprising one or more health-interaction values for each human of the one or more humans;
the graphical user interface comprises:
a map depiction of the monitored physical area with one or more location identifiers of the one or more humans, wherein locations, on the map depiction of the monitored physical area, of the one or more location identifiers are determined based on the location data, and
wherein each location identifier, of the one or more location identifiers, is visually associated with one or more displayed health-interaction values, of the health-interaction data, for a human associated with said each location identifier.

14. The one or more non-transitory computer readable media of Claim 13, wherein:
the location data is real-time location data;
the one or more real-time locations for the one or more respective humans are real-time locations; and
the one or more location identifiers in the map depiction of the monitored physical area reflect the real-time locations for the one or more humans.

15. A computer-executed method comprising:
receiving data from a set of information sources comprising a location information system (140) and a health kiosk information system (150);
wherein said receiving data comprises, for each information source of the set of information sources, receiving data via an application programming interface (API) for said each information source;
generating graphical user interface data which, when processed at a computing device (110), causes a graphical user interface to be displayed that visually aggregates representations of the received data from the set of information sources; and
causing the graphical user interface data to be transmitted to a client computing device (130),
wherein the method is performed by one or more computing devices.
